Europäisches Patentamt

European Patent Office

Office européen des brevets

(11) Publication number: **0 266 459**

**A1**

(12) **EUROPEAN PATENT APPLICATION**

(21) Application number: 86308553.6

(22) Date of filing: 03.11.86

(51) Int. Cl.4 **A61K 37/50** , C12N 9/04 , A61K 39/395 , C12N 15/00 , A61K 43/00 , C12P 21/00 , //(C12P21/00,C12R1:91)

(43) Date of publication of application:
**11.05.88 Bulletin 88/19**

(84) Designated Contracting States:
**AT BE CH DE ES FR GB GR IT LI LU NL SE**

(71) Applicant: **Lakatos, George C.**
**33111 Forrest Apartment 226**
**Wayne Michigan 48184(US)**

(72) Inventor: **Lakatos, George C.**
**33111 Forrest Apartment 226**
**Wayne Michigan 48184(US)**

(74) Representative: **Taylor, Phillip Kenneth et al**
**W.P. THOMPSON & CO. Coopers Building**
**Church Street**
**Liverpool L1 3AB(GB)**

(54) A lactate dehydrogenase or an antibody which reacts with lactate dehydrogenase in human cancer cells for use in the treatment of cancer.

(57) The nature of the present invention is to provide compositions for use in a method of inhibiting the development of cancer cells in humans by restricting the biochemical reaction within these cells in a manner which prevents the spread of the neoplasm and result in necrosis of the cancerous tissue. This object is achieved in accordance with the invention by providing:

(i) A lactate dehydrogenase (LDH) obtained from a primate other than the patient to be treated, for use in the treatment of human cancer patients having cancer cells which include LDH, or

(ii) An antibody which reacts with the lactate dehydrogenase (LDH) in human cancer cells for use in inhibiting the growth of cancer cells in a human cancer patient.

EP 0 266 459 A1

# A LACTATE DEHYDROGENASE OR AN ANTIBODY WHICH REACTS WITH LACTATE DEHYDROGENASE IN HUMAN CANCER CELLS FOR USE IN THE TREATMENT OF CANCER.

The nature of the present invention is to provide compositions for use in a method of inhibiting the development of cancer cells in humans by restricting the biochemical reaction within these cells in a manner which prevents the spread of the neoplasm and result in necrosis of the cancerous tissue. This object is achieved in accordance with the invention by providing:

(i) A lactate dehydrogenase (LDH) obtained from a primate other than the patient to be treated, for use in the treatment of human cancer patients having cancer cells which include LDH, or

(ii) An antibody which reacts with the lactate dehydrogenase (LDH) in human cancer cells for use in inhibiting the growth of cancer cells in a human cancer patient.

In the past most attempts to discover a cure for cancer have involved research directed towards identifying the cause or causes of the malignancy, either generally or specifically. Hence, a broad range of carcinogenic agents have been partially identified. However, very little effective work has been done previously in the area of identifying and inhibiting the basic biochemical reactions involved in the development of malignant neoplasms and metastasis.

It has long been known that the metabolic or energy process for many types of cancer cells in a living body is largely anaerobic, involving glycolysis, i.e. the process by which glucose is converted to lactic acid in the presence of an enzyme lactate dehydrogenase referred to herein as LDH. Without the LDH the lactic acid is not produced. On the other hand, the metabolic process for normal cells in a living body is largely aerobic.

The applicant as long ago as 1956 took interest in the Pathway concept and the possibility that an understanding could make a treatment to cancer available. In his inaugural dissertation "The influence of lactic acid dehydrogenase on malignant tumour tissue" University of Berne, Switzerland, he considered the treatment of tumour cell lines in a series of experiments. In the first he used yeast extracts and in the latter LDH from rabbit muscle, and by direct injection into human and animal neoplastic tissues he examined the effects of these injections, this work being carried out in vivo.

Other prior art is available in the form of a series of papers in Biochemica et Biophysica Acta between 1966-1968.

In the first of these papers Gregory K.F. Ng. C.I.V. and Pastekoek J.F.C. used an antibody to LDH to study the effect of antibodies in the inhibition of glycolysis in cell homogenates of rabbit liver, rabbit VX2 carcinoma, and mouse Ehrlich cell.

The second paper in the series showed the effects of anti-LDH complementary to the animal cells under test in vitro. These results showed that rate of lactic acid formation and protein synthesis by non-malignant embryonic rabbit kidney and muscle cells growing in vitro were not affected by anti-LDH.

The final paper in the series, Ng. et al 1968, showed homologous anti-LDH was taken up and accumulated within rabbit VX2 carcinoma cells and mouse Ehrlich carcinoma cells growing in vitro.

The applicant in a paper, Cancer Research 34, 1395-1400, June 1974, showed that, in the particular case of L1210 leukemia bearing DBA/2 mice, when injected with an isoenzyme to porcine LDH the antibody in some cases was able to produce remissions to this specific lymphatic leukemia. Although one may try to extrapulate these and other results such extrapulation amounts to mere speculation.

The nature of the proposed invention differs in many ways from the prior art available in that:

1) The applicant has proposed the injection into the human cancer patient of LDH from another primate whose LDH is sufficiently dissimilar to the patient's to be recognized as foreign, such that the patient's body will develop antibodies to the foreign LDH, yet is sufficiently similar to the patient's LDH so that the antibodies formed by the patient, though specific to the foreign LDH, will also cross-react with the patient's LDH. As a consequence of such an injection, the LDH mediated glycolysis necessary for growth of the cancer cell is interrupted and the cancer cells are destroyed. Furthermore, as noted in the last paragraph of page 5 of the specification and continuing on page 6, the effect of incorporating the LDH can "reach the very last cancer cell", rendering the present invention useful as an aid to surgery or other processes that remove a major portion of a malignant tumour but fail to reach every last cell.

At the outset, no one as far as the applicant is aware, has ever injected LDH from a monkey or any mammal into a human being for a cancer cure or for any other cure. It has not been recognized heretofore by cancer researchers that cancer cells could be completely destroyed by the injection of a foreign LDH into a cancer patient (to induce the formation of antibodies to the foreign LDH by the patient's body) wherein the foreign LDH is sufficiently similar to the patient's that the antibodies thus formed cross-react with the patient's LDH and starve the patient's cancer cells.

Where the patient's immunological system is not functioning normally and cannot produce antibodies to the foreign LDH a possible treatment can be used wherein the desired antibodies are produced in a host (which may be mammal, primate, or human) and thereafter injected in the patient.

The applicant's invention differs from the prior art in that, the references Gregory et al and Ng. et al, teach the specificity of the antibodies to tumour cells and the inhibiting properties of the antibodies to tumour cell glycolysis. These references are not concerned with the type of antibody that the applicant claims since the applicant teaches the injection of an LDH that is FOREIGN to the patient and Gregory et al produce homologous antibodies, i.e. an anti-mouse LDH antibody is produced by injecting a chicken with mouse LDH and then collecting said antibody or injecting back into the mouse. It is the cross-reactivity referred to by the applicants that is important.

Gregory I states that "the use of homologous anti-LDH preparations to inhibit the glycolysis of normal and malignant rabbit and malignant mouse cell homogenates is reported in the present paper". In the results it states that "no significant differences in inhibition occured with either liver (normal) or tumour homogenates and that the present inhibition obtained with dilute liver (normal) homogenates was greater than that obtained with tumour homogenates".

If this is so it can no way be suggested from the prior art that a cure for cancer could be found since normal cell would be inhibited as much or more than tumour cells.

In Ng. II, Ng. states that anti-LDH inhibited glucose uptake by rabbit tumour cells growing in vitro. However, the antibody was developed in response to rabbit LDH (not an LDH foreign to rabbits). His results are in fact inclonclusive and contradictory as far as a potential cure for cancer is concerned.

Cancer can be induced by a great range of agents, including chemical carginogenesis, such as ionizing radiation. Because of their diversity it is certain these agents must specifically operate by different biochemical routes. However, it appears that these routes lead to a key alteration similar in principle in the different cases, notwithstanding the great diversities between the carcinogens themselves or even between many of the oncogenic viruses.

As has been well known for several decades, the metabolism of malignant tumour cells in the human body and other animal life is different from that of normal cells. A normal cell utilizes both glycolysis and the tricarboxylic acid cycle, the latter requiring the presence of oxygen and produces water and carbon dioxide in the metabolic process. Cancer cells, on the other hand, are largely anaerobic. The energy for the rapid and normally uncontrollable growth of a malignant tumour cell therefore results from glycolysis, the chemical reactions in which glucose is converted to lactic acid. However, the presence of the enzyme lactate dehydrogenase or lactic acid dehydrogenase (hereinafter usually referred to as LDH in accordance with conventional usage) is essential for the completion of the reactions and the production of lactic acid. The LDH functions as a catalyst, and lactic acid will not be produced in the absence of this enzyme.

All energy production in mammalian tissue involves two metabolic pathways. One is the glycolytic cycle, the enzymes for which reside in the cell cytoplasm, and the other is the tricarboxylic acid cycle whose enzymes are organized within in the cell mitochondria. Under normal conditions glycolysis proceeds in the cytoplasm of the cell to the pyruvate state. The pyruvate produced is then fed, via acetyl coenzyme A, into the tricarboxylic acid cycle, while the resultant reduced form nicotinamide adenine dinucleotide (NADH) is reoxidized via the electron transport chain.

In the event the mitochondrial mechanisms are not available, pyruvate may be reduced to lactate in the cytosol, thus reoxidizing the NADH formed earlier in the glycolytic cycle. This reaction is mediated by lactate dehydrogenase. Many cancer cells, for reasons not fully understood at the present time, are dependent on glycolysis, rather than on the tricarboxylic acid cycle and the associated electron transport chain, for their energy production. It has now been found that these cancer cells may be destroyed by inhibiting the LDH - mediated step of glycolysis because the vital coenzyme, nicotinamide adenine dinucleotide in oxidized form, will be rapidly depleted. The anaerobic energy-yielding conversion of glucose to lactic acid thus is not completed since the reaction appears to be halted at the pyruvic acid stage.

In accordance with the procedure involved in the present invention the human cancer patient is immunised with LDH. This LDH is preferably obtained from a primate other than the patient to be treated, preferably monkeys and the family Pongidae. Hence the patient responds by producing antibodies to the foreign LDH "in situ". These antibodies may be to particular units of the LDH molecules called isoenzyme or more specifically to particular regions namely haptens. The LDH obtained from the higher primates, while functionally "recognised" as a foreign protien by the cancer host, is structurally sufficiently similar to the human LDH so that the antibody (Anti LDH) formed can cross-react with the human LDH, thus inhibiting the human cancer LDH activity and thereby destroying the cancer cells.

In some instances it appears desirable to treat patients by passive rather than active immunization because frequently the human cancer patient is incapable of producing the necessary antibodies. Therefore, passive immunization is indicated in instances where the patient is severely debilitated.

In a preferred embodiment of the process of the present invention, an anti-LDH derived from another human or other primate is administered to the cancer patient rather than injecting primate LDH. The anti-LDH is "raised" in the primate, the primate thereafter bled, and the anti-LDH in said blood isolated from other plasma proteins by chromatography. A usable variation of this procedure invloves obtaining LDH from a rhesus monkey, for example, injection it into another primate to develop antibodies, and injecting the anti-LDH so obtained into the human cancer patient.

This process also can utilize a transfer mechanism in which the anti-LDH produced in a healthy human body, for example, are made radioactive and then introduced parenterally into the body of the patient to destroy the cancer cells by radioactive means, the anti-LDH functioning as the delivery vehicle for the radionuclide. Such a radionuclide can be attached to a protein such as the anti-LDH by the Hunter Greenwood procedure. Alternatively, it is possible to use the aforesaid transfer mechanism by producing the anti-LDH in the body of the cancer patient and reintroducing this anti-LDH into the patient's body parenterally.

In accordance with the principles involved in the present invention, it has been recognized that malignant tumor tissue breaks down glucose to lactic acid at a far greater rate than does normal tissue. Hence cancer cells have significantly greater susceptibility to inhibition by the anti-LDH than do normal cells, making the use of LDH in the process of this invention especially effective. Moreover, immunological regimens such as that involved in the present invention can reach the very last cancer cell. This action is in contrast to most chemotherapeutical regimens which are effective in reducing the number of cells in a malignant tumour but evidence an inability to affect every such cell. Also of clinical importance is the fact that the administered agent, LDH, is non-toxic. Furthermore, in the event of a cancer patient who was previously treated in accordance with the process of the present invention subsequently develops a new cancer, even one of a different type, treatment is simplified because the injection of LDH from the source previously employed produces an amnestic response which is characteristic of immunological responses. This reaction, in turn, indicates the potential practical applicability of the inventive concept as a vaccination procedure. There is no reason why the LDH process described herein cannot be used in conjunction with chemotherapy, surgery, radioisotope therapy and perhaps other forms of immunotherapy.

The LDH may be obtained from a rhesus monkey, for example, or other desired primate by standard procedures such as those described on pages 441-443 of the article by Arthur Kornberg entitled "Lactic Dehydrogenase of Muscle" which appeared in Methods of Enzymology, Vol. I, Ed: Colowick Kaplan. Academic Press, 1955.

Purification of the LDH produced can be achieved by standard chromatographic procedures, such as affinity chromatography or ion exchange chromatography. The purified material is then dialyzed and subsequently lyophilized for storage purposes. When it is to be employed in immunization, the lyophilized material is thereafter redissolved in water.

While the present invention has been described by means of certain specific examples, it shoud be understood that additional variations of the procedures embodying the inventive concept are possible and that the scope of the invention is set forth in the following claims.

For example, a basic concept of the treatment described herein comprises the selective inhibition of LDH mediated glycolysis in cancer cells of a human patient by providing antibodies in the patient that will react with the LDH associated with the cancer cell and essential for the glycolysis therein, thereby to inhibit the LDH mediated glycolysis. LDH derived from primate (including human) tissue comprises a spectrum of LDH types or variatons of isozymes, such as LDH-1, 2, 3, 4, 5, & K for example, and possibly others not even known, which when injected parenterally into a cancer patient in accordance with the procedure described above induces the patient's immune system to produce a corresponding spectrum of anti LDH antibodies - referred to herein simply as LDH antibodies.

Some of the LDH In the aforesaid spectrum exist in large quantities in malignant tumours and mediate the anaerobic glycolysis required for growth of the cancer cells. Likewise, some of the antibodies in the spectrum will react with the LDH required for the anaerobic glycolysis and essentially starve the cancer cells. The LDH and antibody spectrum have been employed with positive results in the treatment of cancer infected mice (see applicant's co-authored paper referred to above) and terminal human cancer patients (see copy of applicant's February 28, 1985 Affidavit in the file of copending application S.N. 774,242, filed August 13, 1985) regardless that a human cancer patient considered terminal, i.e., refractory to conventional treatment and having a life expectancy of only a few weeks or at most a few months, usually has a seriously impaired immune system.

Specific types of LDH, such as LHD-5 and LDH-K, are appreciably more abundant in cancerous tumours than in normal tissue and are essential to the growth of a cancer cell. Accordingly, a preferred cancer treatment within the concept described herein uses antibodies to such specific LDH types. The treatment of a cancer patient is enhanced especially by providing in the patient antibodies to LDH-5 and LDH-K known to be essential to the metabolic process of the cancer cell, either by stimulating the patient's immune system to develop the antibodies, or by injecting the antibodies directly.

Further, by way of example, LDH associated with and essential for the growth of any specific type of cancer may be extracted from the cancer, e.g. the patient's cancer. Antibodies thereto may then be developed by conventional procedures, such as in a host animal as described herein, and thereafter injected into the patient. Once an antibody is isolated, it may be cloned in quantity by known methods.

LDH-K is rare if it exists at all in normal cells growing in an oxygenated environment but it is associated in large concentrations with many types of cancer cells and is essential to their anerobic metabolism. Accordingly, LDH-K may be isolated from cancerous tissue by known procedures and antibodies thereto may be developed for injection into cancer patients as described herein to inhibit anerobic glycolysis in the cancer cells. By reason of the specificity of such antibodies to LDH-K, they will have no observable effect on normal cells. Accordingly, such antibodies to LDH-K may also be employed to transport cytotoxic or irradiated drugs or other cell destroying substances, such as used in conventional chemotherapy or radioisotope therapy, directly into the cancer tumours without significantly affecting adjacent normal cells.

In addition to LDH-K, the LDH extracted from cancerous tissue usually comprises large amounts of LDH-5, as compared to the LDH-5 ordinarily associated with normal tissue, and possibly an LDH-5,K complex. Preferably these isozymes that exist in large quantities in cancerous tissue and are essential for metabolism of the cancer cell are extracted from cancerous tissue. Antibodies specific to these LDH isozymes are then developed and injected into the cancer patient. Such antibodies recognize and react directly with the patient's LDH isozymes to which they are specific and inhibit the LDH function necessary for completion of the metabolic cycle of the cancer cell. The result is necrosis of the patient's cancer cells without reliance upon a cross reaction effect.

Although the various LDH isozymes existing in all types of human cancers have not been studied in detail, a preponderance of LDH-K has been found in most human carcinomas, as compared to adjacent normal or non-tumour tissue. (Anderson et al U.S.Patent No. 4,558,007). Usually, the more active the cancer, the greater the concentration of the LDH-K, such that the quantity of LDH-K in cancer serum is a measure of cancer cell metabolism. Another predominant LDH isozyme found in cancerous tissue is LDH-5. Accordingly in a preferred treatment, the more prevalent LDH isozymes which may be LDH-5, LDH-K and and LDH-5,K complex, are extracted from human cancer tissue, which can be the patient's cancer. Each type of isozyme is maintained separately from the others and is injected into a mammal, such as a mouse or rat selected to produce antibodies specific to the type of injected isozyme. The immune system of the mammal produces antibodies to the injected isozyme, as for example, in the mammal's spleen. The mammal is sacrificed and the cells producing the anti-LDH antibody developed by the mammal are extracted from the spleen and thereafter cultivated in quantity by conventional procedures.

Each antibody producing cell will reproduce itself under known controlled conditions but such reproduction is slow. Thus one of several other conventional procedures for multiplying these cells is preferalby employed. In one such procedure, a number of these cells are extracted from the spleen. Each cell is spliced or fused with a separate myeloma cell to produce a hybridoma that can be rapidly grown or multiplied conventionally in a culture. Each hybridoma is then multiplied separately from the others to produce a separate batch of that particular hybridoma. Each batch of hybridoma is maintained separately from the others and is "washed" by conventional procedure to remove the hybridoma cell structure and leave only a batch of antibodies.

Each batch of antibodies is then tested in turn in vitro against the human malignant tumour to determine which batch is most effective in blocking the anerobic metabolic process of cancer cells. The antibodies from the most effective batch may then be cloned in quantity for subsequent parenteral injection into the cancer patient and even directly into the cancerous tumour when feasible. By virtue of the specificity of the injected antibody, it will react with the corresponding LDH ( to which it is specific) of cancer cells throughout the body of the patient without observable effect on normal tissue.

Also as explained more generally above, the antibodies obtained by the foregoing process may be tagged with cytotoxic drugs, radioisotopes, or other cell destroying substances to effect a two-pronged attack against particularly active cancers without excessive injury to adjacent normal tissue.

## Claims

1. A lactate dehydrogenase (LDH) obtained from a primate other than the patient to be treated, for use in the treatment of human cancer patients having cancer cells which include lactate dehydrogenase.

2. The use of LDH as claimed in claim 1, for the manufacture in situ of antibodies for use in the treatment of human cancer patients having cancer cells which include LDH.

3. An antibody which reacts with the lactate dehydrogenase (LDH) in human cancer cells for use in inhibiting the growth of cancer cells in a human cancer patient.

4. An antibody as claimed in claim 1, characterised in that it has been obtained by injecting LDH extracted from a primate into a mammal and isolating the antibody to said LDH produced in said mammal.

5. An antibody as claimed in claim 3, characterised in that said antibody has been obtained by a procedure which comprises injecting into a mammal LDH extracted from a primate, isolating from said mammal a number of cells of said mammal which are producing antibodies to said LDH, forming from each of said cells a hybridoma capable of rapid multiplication, effecting separate multiplication of each of said hybridoma cells to produce separate batches of hybridoma cells, isolating the monoclonal LDH antibody form each of said batches and testing each isolated monoclonal LDH antibody against cancerous tissue to determine the most effective of said monoclonal LDH antibodies and cloning the antibodies corresponding to said most effective monoclonal LDH antibody in quantity.

6. An antibody as claimed in any of the preceding claims, characterised in that said LDH is obtained from human cancer tissue.

7. An antibody as claimed in claim 6, wherein the human cancer tissue is obtained from the patient to be treated.

8. An antibody as claimed in any of the preceding claims, characterised in that said antibody is associated with a cell-destroying agent which destroys cancer cells.

9. An antibody as claimed in claim 8, characterised in that said cell-destroying agent is selected from cytotoxic and radio-active materials.

10. An antibody as claimed in any of the preceding claims, characterised in that it is an antibody which reacts with an LDH selected from LDH-5 or LDH-K.

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | CHEMICAL ABSTRACTS, vol. 69, no. 19, 4th November 1968, page 6909, abstract no. 74069a, Columbus, Ohio, US; D. DENNIS: "Use of specific macromolecular complexing agents for enzyme purification: beef heart dehydrogenase", & ANAL. BIOCHEM. 1968, 24, 541-4 * Abstract * | 1 | A 61 K 37/50<br>C 12 N 9/04<br>A 61 K 39/395<br>C 12 N 15/00<br>A 61 K 43/00<br>C 12 P 21/00 //<br>(C 12 P 21/00<br>C 12 R 1:91 ) |
| | --- | | |
| X | BIOLOGICAL ABSTRACTS, vol. 63, no. 11, 1977, page 6278, abstract no. 63930; D. POSTELNICU et al.: "Influence of anti-lactate dehydrogenase M antibodies on the proliferation of KB tumor cells", & C R SEANCES SOC. BIOL. FIL. 170(3), 537-543, 1976 * Abstract * | 1-4 | |
| | --- | | TECHNICAL FIELDS SEARCHED (Int. Cl.4) |
| X | FR-A- 898 671 (SCHERING AG) * Page 1, lines 19-31; page 2, lines 8-32 * | 1-4 | A 61 K<br>C 12 P |
| | --- -/- | | |

The present search report has been drawn up for all claims

| Place of search | Date of completion of the search | Examiner |
|---|---|---|
| THE HAGUE | 09-07-1987 | FERNANDEZ Y BRANAS F |

## DOCUMENTS CONSIDERED TO BE RELEVANT

Page  2

| Category | Citation of document with indication, where appropriate, of relevant passages | Relevant to claim | CLASSIFICATION OF THE APPLICATION (Int Cl 4) |
|---|---|---|---|
| A | CHEMICAL ABSTRACTS, vol. 77, no. 15, 9th October 1972, page 12, abstract no. 96787a, Columbus, Ohio, US; B.S. SAMAGH et al.: "Antibody to lactate dehydrogenase. V. Use as a carrier for introducing diphtheria toxin into mouse tumor cells", & BIOCHIM. BIOPHYS. ACTA 1972, 273(1), 188-98 * Abstract * | 1-4,6-10 | |
| | --- | | |
| A | CHEMICAL ABSTRACTS, vol. 99, no. 23, 5th December 1983, page 380, abstract no. 190584h, Columbus, Ohio, US; R.E. GOLDMAN-LEIKIN: "Monoclonal antibodies for immunochemical analysis of mammalian lactate dehydrogenases-C4", & DISS. ABSTR. INT. B 1983, 44(3), 676 * Abstract * | 5 | TECHNICAL FIELDS SEARCHED (Int. Cl 4) |
| | --- | | |
| A,D | CHEMICAL ABSTRACTS, vol. 81, no. 11, 16th September 1974, page 349, abstract no. 62038z, Columbus, Ohio, US; G. LAKATOS et al.: "Prolonged remissions of lymphatic leukemia in DBA/2 mice induced with endogenously produced lactate dehydrogenase antibody", & CANCER RES. 1974, 34(6), 1395-400 | | |
| | ---   -/- | | |

The present search report has been drawn up for all claims

| Place of search | Date of completion of the search | Examiner |
|---|---|---|
| THE HAGUE | 09-07-1987 | FERNANDEZ Y BRANAS F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

## DOCUMENTS CONSIDERED TO BE RELEVANT

Page  3

| Category | Citation of document with indication, where appropriate, of relevant passages | Relevant to claim | CLASSIFICATION OF THE APPLICATION (Int. Cl 4) |
|---|---|---|---|
| A | CHEMICAL ABSTRACTS, vol. 87, no. 19, 7th November 1977, page 418, abstract no. 150045d, Columbus, Ohio, US; A.O. GRUBB: "Demonstration of circulating IgG-lactate dehydrogenase immune complexes by crossed immunoelectrophoresis", & SCAND. J. IMMUNOL. SUPPL. 1975, 2(Quant. Immuno-Electrophor.) 53-7 | | |

-----

TECHNICAL FIELDS
SEARCHED (Int. Cl.4)

The present search report has been drawn up for all claims

| Place of search | Date of completion of the search | Examiner |
|---|---|---|
| THE HAGUE | 09-07-1987 | FERNANDEZ Y BRANAS E |